# EUROPÄISCHE PATENTSCHRIFT

(11) **EP 1 301 185 B1**
(45) Veröffentlichungstag und Bekanntmachung des Hinweises auf die Patenterteilung: **28.10.2009**
(21) Anmeldenummer: 01947397.4
(22) Anmeldetag: 25.06.2001
(51) Int. Cl.: A61K 31/445, A61K 31/325, A61P 33/10

(54) **ANTIHELMINTIKA ZUR VERHINDERUNG VON PARASITÄREN INFEKTIONEN BEI MENSCH UND TIER**
ANTHELMINTHIC AGENTS FOR THE PREVENTION OF PARASITIC INFECTIONS IN HUMANS AND ANIMALS
PRODUITS ANTHELMINTIQUES PERMETTANT DE PREVENIR LES INFECTIONS PARASITAIRES CHEZ L'HOMME ET L'ANIMAL

(30) Priorität: 06.07.2000 DE 10032878
(43) Veröffentlichungstag der Anmeldung: 16.04.2003
(73) Patentinhaber: Saltigo GmbH, 40764 Langenfeld (DE)
(72) Erfinder: HARDER, Achim, 51109 Köln (DE); VON SAMSON-HIMMELSTJERNA, Georg, 30900 Mellendorf (DE); KRÜGER, Bernd-Wieland, 51467 Bergisch Gladbach (DE); MEHLHORN, Heinz, 41468 Neuss (DE); SCHMIDT, Jürgen, 40221 Düsseldorf (DE)
(74) Vertreter: Wichmann, Birgid
(86) Internationale Anmeldenummer: PCT/EP2001/007201
(87) Internationale Veröffentlichungsnummer: WO 2002/002115

(56) Entgegenhaltungen:
- EP-A- 0 289 842
- EP-A- 0 300 189

## Beschreibung

Die vorliegende Erfindung betrifft Mittel enthaltend bestimmte, als Repellentien geeignete Wirkstoffe und deren Verwendung zur Verhinderung einer Infektion des Menschen bzw. von Tieren mit den Infektionsstadien von parasitischen Plattwürmern (Plathelminthen). Die Mittel kommen dabei auf der Haut gegen solche Stadien der Plattwürmer, sogenannte Cercarien, zum Einsatz, die durch die Haut in den Wirtskörper eindringen können.

Mehrere Arten von Plathelminthen verursachen schwerwiegende Erkrankungen von Menschen und Tieren. In tropischen Ländern führen insbesondere Infektionen mit Schistosoma-Arten zu chronischem Leiden und oft zum Tod. Wichtige Erreger sind Schistosoma mansoni, Schistosoma haematobium und Schistosoma japonicum. Betroffen sind die einheimische Bevölkerung, Touristen, Mitarbeiter von humanitären Hilfsorganisationen sowie militärisches Personal. Bei der Infektion des Menschen können die infektionsfähigen Cercarien, die sich im Wasser offener Gewässer befinden, durch die Haut in den Körper eindringen.

Ebenfalls problematisch ist in Ländern mit gemäßigtem Klima der Befall von Menschen mit Cercarien verschiedener Arten der Gattungen Trichobilharzia und Ornithobilharzia, die sich in die Haut einbohren und eine Dermatitis hervorrufen können. Solche Infekte erfolgen bei Freizeitaktivitäten an Binnengewässern oder Meeresküsten sowie bei Tätigkeiten in der Fischerei, Teichwirtschaft oder Feldbewässerung. Generell ist in vielen Situationen des täglichen Lebens der Kontakt der Haut mit u. U. kontaminiertem/infiziertem Wasser unvermeidbar.

Eine erfindungsgemäße Vorbehandlung der Haut mit anthelminthisch wirkenden Substanzen kann jedoch vor einem Eindringen der Erreger schützen.

In der Vergangenheit wurden bereits einige Verbindungen auf ihre Tauglichkeit zur Verhinderung von Infektionen mit solchen Parasiten getestet. Die bisher für die erfindungsgemäßen Zwecke beschriebenen Substanzen sind jedoch toxisch, wenn sie durch die Haut oder nach oraler Aufnahme in den Körper gelangen:

So zeigt z.B. Hexachlorophen eine abtötende Wirkung auf Cercarien von Schistosoma mansoni (Fripp, P. J. and Armstrong, F. I., The efficacy of hexachlorophene skin cleanser as a cercariae repellent. South African Med. J. 47: 1973, 526-527). Hexachlorophen kann wegen gesundheitlicher Risiken, insbesondere Leberschäden, beim Menschen nicht auf der Haut angewandt werden. Es ist giftig bei Berührung mit der Haut und beim Verschlucken, kann möglicherweise Missbildungen hervorrufen und ist eventuell krebserregend [Kommission der Europäischen Gemeinschaften, Richtlinie 93/72/EWG vom 1. Sept. 1993, Anhang Bd. I und II (EU Gefahrstoff-Verordnung) mit Ergänzungen bis 1999, Amtsblatt der EUL258A, 36. Jahrgang, 16. Okt. 1993, Ergänzungen bis 1997].

Niclosamid wirkt gegen Eindringen von Cercarien [Bruce, J. I. et al. (1992) Efficacy of niclosamide as a potential topical antipenetrant (TAP) against cercariae of Schistosoma mansoni in monkeys. Mem. Inst. Oswaldo Cruz 87:28 1-289.] ist aber toxikologisch bedenklich, weil es möglicherweise vererbbare genetische Schäden verursachen kann (Registry of Toxic Effects of Chemical Substances, National Institute of Occupational Safety and Health). Die Anwendung auf der Haut bei Exposition in Gewässern verbietet sich durch seine umweltgefährdende Eigenschaft, da Niclosamid stark wassergefährdend ist [Umweltbundesamt (Hrsg.) Katalog wassergefährdender Stoffe. LTwS-Nr. 12. Mai 1996 mit laufenden Ergänzungen, Berlin 1996]. Daher hat bisher keine kommerzielle Anwendung gegen Cercarien beim Menschen stattgefunden.

N, N-Diethyl-m-toluamid (DEET) wirkt auf Cercarien von Schistosoma mansoni [Salafsky, B. et al. Evaluation of N, N-diethyl-m-toluamide (DEET)as a topical agent for preventing skin penetration by cercariae of Schistosoma mansoni. Am. J. Trop. Med. Hyg. 58: 1998, 828- 834). DEET besitzt jedoch einige ungünstige Eigenschaften.

Die Wirkung der bisher beschriebenen Anthelminthika gegen infektiöse Stadien von Plathelminthen wurde bisher nur an Cercarien der Art Schistosoma mansoni getestet, so dass eine Wirksamkeit dieser Mittel gegen andere Wurmarten bislang nicht nachgewiesen war.

Es wurde nun überraschenderweise gefunden, dass die erfindungsgemäßen Mittel geeignet sind, um Mensch und Tier einen effektiven Schutz vor Infektionen mit Plathelminthen, insbesondere Schistosoma haematobium, Schistosoma japonicum, Trichobilharzia spp. und Ornithobilharzia spp., aber auch Echinostoma spp. u.a. zu bieten.

Die Erfindung betrifft demnach
1. Mittel zur Anwendung in einem Verfahren zur Abwehr von helminthischen Parasiten, gekennzeichnet durch einen Gehalt an mindestens einer Verbindung der Formel (I) 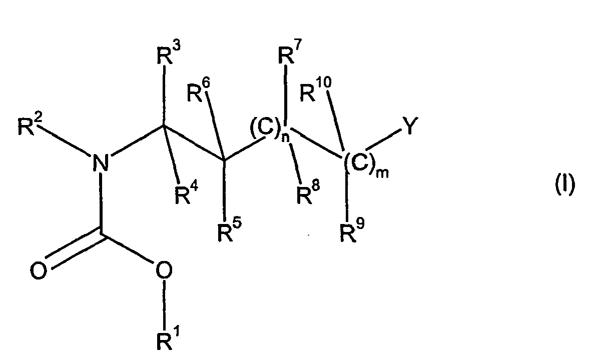 in welcher
   - Y: für Wasserstoff, gegebenenfalls substituiertes Alkyl oder für den Rest O-X steht,
   - X: für Wasserstoff, COR¹¹, COOR¹² oder R¹³ steht,
   - R¹: für einen gegebenenfalls substituierten Alkyl-, Alkenyl-, Alkinyl-, Cycloalkyl- oder Cycloalkenylrest steht,
   - R², R¹¹, R¹² und R¹³: gleich oder verschieden sind und für gegebenenfalls substituierte Alkyl- oder Alkenylreste stehen,
   - R³ bis R¹⁰: gleich oder verschieden sind und für Wasserstoff oder für gegebenenfalls substituierte Alkylreste stehen und wobei R² und R³ oder R³ und R⁷ oder R³ und R⁵ oder R⁵ und R⁷ gemeinsam mit den Atomen, an welche sie gebunden sind, auch einen gegebenenfalls substituierten monocyclischen Ring bilden können
   und
   - n und m: gleich oder verschieden sind und 0 oder 1 bedeuten.
2. Mittel zur Anwendung in einem Verfahren zur Abwehr von helminthischen Parasiten gemäß Punkt 1, **dadurch gekennzeichnet, dass** sie mindestens eine Verbindung der Formel (I) enthalten,
   in welcher
   - Y: für Wasserstoff, C₁-C₆-Alkyl oder für den Rest O-X steht,
   - X: für Wasserstoff, COR¹¹ oder R¹³ steht,
   - R¹: für C₃-C₇-Cycloalkyl, C₃-C₇-Cycloalkenyl, C₁-C₂-Alkyl-C₃-C₇-Cycloalkyl, C₁-C₂-Alkyl-C₃-C₇-Cycloalkenyl, wobei die Cycloalkyl- oder Cycloalkenylringe der vorgenannten Reste gegebenenfalls bis zu dreifach durch C₁-C₆-Alkyl oder durch eine C₁-C₆-Dialkylenbrücke substituiert sind, oder
   - R¹: für C₁-C₇-Alkyl, C₂-C₇-Alkenyl oder C₂-C₇-Alkinyl steht,
   - R², R¹¹, R¹³: gleich oder verschieden sind und für C₁-C₆-Alkyl stehen,
   - R³ bis R⁸: gleich oder verschieden sind und für Wasserstoff oder C₁-C₆-Alkyl stehen, wobei R² und R³ oder R³ und R⁷ oder R³ und R⁵ oder R⁵ und R⁷ gemeinsam mit den Atomen, an welche sie gebunden sind, auch einen 5-oder 6-gliedrigen monocyclischen Ring bilden können und
   - n: für 1 und m für 0 steht.
3. Verwendung von Verbindungen der Formel (I) gemäß Punkt 1 für die Herstellung von Mitteln zur Abwher von helminthischen Parasiten.
4. Verwendung nach Punkt 3, **dadurch gekennzeichnet, dass** man Verbindungen der Formel (I), gemäß Anspruch 1 mit Streckmitteln und/oder oberflächenaktiven Mitteln vermischt.

Gemäß einer bevorzugten Ausführungsform steht in Formel (I) der Substituent Y für Wasserstoff oder C₁-C₆-Alkyl, wie z. B. Methyl, Ethyl, Propyl, Isopropyl, n-Butyl, sek.-Butyl, iso-Butyl oder tert.-Butyl, Pentyl oder Hexyl. In diesem Fall steht R¹ bevorzugt für C₃-C₇-Cycloalkyl, C₃-C₇-Cycloalkenyl, C₁-C₂-Alkyl-C₃-C₇-Cycloalkyl, C₁-C₂-Alkyl-C₃-C₇-Cycloalkenyl, wobei die Cycloalkyl- oder Cycloalkenylringe der vorgenannten Reste gegebenenfalls bis zu dreifach durch C₁-C₆-Alkyl oder durch eine C₁-C₆-Dialkylenbrücke substituiert sind.

Gemäß einer weiteren Ausführungsform werden in den erfindungsgemäßen Mitteln Verbindungen der Formel (I) eingesetzt, in welcher
- Y: für den Rest O-X steht,
- X: für Wasserstoff, COR¹¹, COOR¹² oder R¹³ steht,
- R¹: für gegebenenfalls substituierte Alkyl-, Cycloalkyl-, Alkenyl- oder Alkinylreste steht,
- R², R¹¹, R¹² und R¹³: gleich oder verschieden sind und für gegebenenfalls substituierte Alkyl- oder Alkenylreste stehen,
- R³ bis R¹⁰: gleich oder verschieden sind und für Wasserstoff oder für gegebenenfalls substituierte Alkylreste stehen und wobei R² und R³ oder R³ und R⁷ oder R³ und R⁵ oder R⁵ und R⁷ gemeinsam mit den Atomen, an welche sie gebunden sind, auch einen gegebenenfalls substituierten monocyclischen Ring bilden können
und
- n und m: gleich oder verschieden sind und 0 oder 1 bedeuten.

Von diesen sind bevorzugt die Verbindungen der Formel (I), in welcher
- X: für Wasserstoff, COR¹¹ oder R¹³ steht,
- R¹: für C₁-C₇-Alkyl, C₃-C₇-Cycloalkyl, C₂-C₇-Alkenyl oder C₂-C₇-Alkinyl steht,
- R², R¹¹, R¹³: gleich oder verschieden sind und für C₁-C₆-Alkyl stehen,
- R³-R⁸: gleich oder verschieden sind und für Wasserstoff oder C₁-C₆-Alkyl stehen, wobei R² und R³ oder R³ und R⁷ oder R³ und R⁵ oder R⁵ und R⁷ gemeinsam mit den Atomen, an welche sie gebunden sind, auch einen 5-oder 6-gliedrigen monocyclischen Ring bilden können und
- n: für 1 und m für 0 steht,

Von diesen besonders bevorzugt sind Verbindungen der allgemeinen Formel (I),
in welcher
- X: für Wasserstoff oder R¹³ steht,
wobei R¹³ für C₁-C₆-Alkyl steht,
- R¹: für C₁-C₇-Alkyl oder C₃-C₇-Alkenyl steht,
- R⁴ bis R⁸: gleich oder verschieden sind und für Wasserstoff oder C₁-C₆-Alkyl stehen,
- R² und R³: gemeinsam mit den Atomen, an welche sie gebunden sind, einen 5- oder 6-gliedrigen monocyclischen Ring bilden,
- n: für 1 und
- m: für 0 steht.

Weiterhin werden von den Verbindungen, in denen Y für den Rest O-X steht, solche bevorzugt, in denen R¹ für C₁-C₇-Alkyl, C₃-C₇-Cycloalkyl oder C₃-C₇-Alkenyl steht, X für COR¹¹ oder R¹³ steht, R² und R¹¹ gleich oder verschieden sind und für C₁-C₆-Alkyl stehen, R³ bis R⁸ gleich oder verschieden sind und für Wasserstoff oder C₁-C₆-Alkyl stehen, R¹³ für C₁-C₆-Alkyl steht, und n für 1 und m für 0 steht.

Ganz besonders zur Verwendung in den erfindungsgemäßen Mitteln bevorzugt werden die Verbindungen der allgemeinen Formel (I), in welcher
- Y: für den Rest O-X steht,
m = 0 und n =1 ist,
- R¹: für C₁-C₄-Alkyl oder C₅-C₆-Cycloalkyl steht,
- R², R¹¹ und R¹³: gleich oder verschieden sind und für C₁-C₆-Alkyl stehen, R³ bis R⁸ für Wasserstoff stehen und X für Wasserstoff, COR¹¹ oder R¹³ steht, wobei R¹¹ und R¹³ die vorgenannte Bedeutung haben.

Weiterhin werden ganz besonders bevorzugt zur Verwendung in den erfindungsgemäßen Mitteln Verbindungen der allgemeinen Formel (I) eingesetzt, in welcher m = 0 und n = 1 ist, R¹ für C₁-C₄-Alkyl steht, R² und R³ gemeinsam mit den Atomen, an die sie gebunden sind, einen 6-gliedrigen Piperazinring bilden, R⁴ bis R⁸ für Wasserstoff steht und X für Wasserstoff oder R¹³ steht, wobei R¹³ für C₁-C₄-Alkyl steht.

Als Beispiele für Verbindungen in denen R² und R³ gemeinsam mit den Atomen, an welche sie gebunden sind, einen gegebenenfalls substituierten monocyclischen Ring bilden, seien die folgenden genannt:

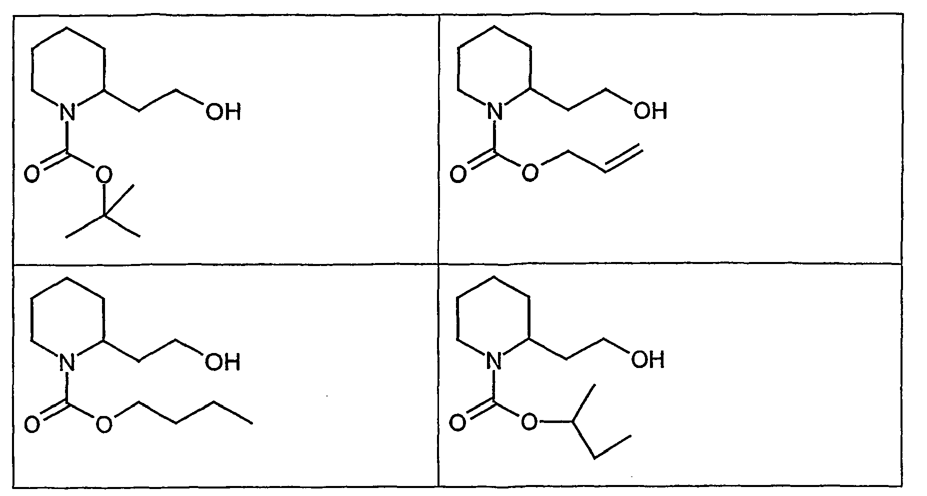

Als Beispiele für Verbindungen, in denen R² und R³ keinen Ring bilden, seien die folgenden genannt:

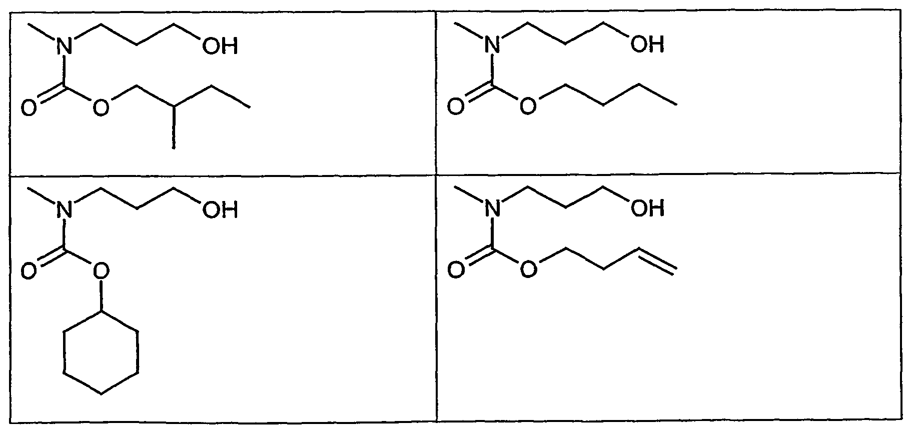

Die Verbindungen der allgemeinen Formel (I) sind entweder bekannt oder können nach bekannten Methoden und Verfahren hergestellt werden (vergl., z. B. Cesare Ferri, Reaktionen der organischen Synthese, Georg Thieme Verlag Stuttgart, 1978, S. 223 und S, 450, sowie EP A 0 289 842).

Die in den erfindungsgemäßen Mitteln enthaltenen Wirkstoffe wurden bereits speziell zur Verwendung als Repellent auf der Haut gegen Insekten und Zecken eingesetzt.

Ein wesentlicher Vorteil der Verwendung der erfindungsgemäßen Verbindungen ist deren hohe Haut-, Pflanzen- und Umweltverträglichkeit und die generell geringe Toxizität dieser Verbindungen.

Es ist weiterhin wünschenswert, beim Aufenthalt im Freien gegen Moskitos geschützt zu sein, die zum einen als belästigend empfunden werden, zum anderen können die Moskitos mit ihren Stichen speziell in den Tropen Krankheiten wie Malaria, verschiedene Viren, Filarien und Parasiten übertragen. Die erfindungsgemäßen Mittel ermöglichen nun die gleichzeitige Prävention vor Infektionen mit Plathelminthen und Schutz vor Moskitos mit einem Mittel. Damit wird die Notwendigkeit der gleichzeitigen Anwendung von zwei verschiedenen, möglicherweise nicht miteinander verträglichen Mitteln auf der Haut vermieden.

Die erfindungsgemäßen Mittel können neben den Wirkstoffen auch alle üblichen Hilfs- und Zusatzstoffe enthalten, welche in Formulierungen zur topikalen Applikation verwendet werden.

Die Anwendung der Wirkstoffe erfolgt direkt oder in Form von geeigneten Zubereitungen dermal oder mit Hilfe wirkstoffhaltiger Formkörper wie z.B. Streifen, Platten, Bänder, Halsbänder, Ohrmarken, Gliedmaßenbänder, Markierungsvorrichtungen.

Die dermale Anwendung geschieht z.B. in Form des Badens, Tauchens (Dippen), Sprühens (Sprayen), Aufgießens (pour-on oder spot-on), Waschens, Schamponierens, Begießens, Einpuderns.

Geeignete Zubereitungen sind:
Lösungen oder Konzentrate zur Verabreichung nach Verdünnung, Lösungen zum Gebrauch auf der Haut, Aufgußformulierungen, Gele;
Emulsionen und Suspensionen zur dermalen Anwendung sowie halbfeste Zubereitungen;
Formulierungen, bei denen der Wirkstoff in einer Salbengrundlage oder in einer Öl in
Wasser oder Wasser in Öl Emulsionsgrundlage verarbeitet ist;

Feste Zubereitungen wie Pulver, wirkstoffhaltige Formkörper.

Lösungen zum Gebrauch auf der Haut werden aufgeträufelt, aufgestrichen, eingerieben, aufgespritzt, aufgesprüht oder durch Tauchen (Dippen), Baden oder Waschen aufgebracht.

Die Lösungen werden hergestellt, indem der Wirkstoff in einem geeigneten Lösungsmittel gelöst wird und evtl. Zusätze wie Lösungsvermittler, Säuren, Basen, Puffersalze, Antioxidantien, Konservierungsmittel zugefügt werden.

Als Lösungsmittel seien genannt: Physiologisch verträgliche Lösungsmittel wie Wasser, Alkohole wie Ethanol, Butanol, Benzylakohol, Glycerin, Kohlenwasserstoffe, Propylenglykol, Polyethylenglykole, N-Methyl-pyrrolidon, sowie Gemische derselben.

Die Wirkstoffe lassen sich gegebenenfalls auch in physiologisch verträglichen pflanzlichen oder synthetischen Ölen lösen.

Als Lösungsvermittler seien genannt: Lösungsmittel, die die Lösung des Wirkstoffs im Hauptlösungsmittel fördern oder sein Ausfallen verhindern. Beispiele sind Polyvinylpyrrolidon, polyoxyethyliertes Rhizinusöl, polyoxyethylierte Sorbitanester.

Konservierungsmittel sind: Benzylalkohol, Trichlorbutanol, p-Hydroxybenzoesäureester, n-Butanol.

Es kann vorteilhaft sein, bei der Herstellung der Lösungen Verdickungsmittel zuzufügen. Verdickungsmittel sind: Anorganische Verdickungsmittel wie Bentonite, kolloidale Kieselsäure, Aluminiummonostearat, organische Verdickungsmittel wie Cellulosederivate, Polyvinylalkohole und deren Copolymere, Acrylate und Methacrylate.

Gele, die auf die Haut aufgetragen oder aufgestrichen werden, werden hergestellt indem Lösungen, die wie oben beschrieben hergestellt worden sind, mit soviel Verdickungsmittel versetzt werden, dass eine klare Masse mit salbenartiger Konsistenz entsteht. Als Verdickungsmittel werden die weiter oben angegebenen Verdickungsmittel eingesetzt.

Aufgießformulierungen werden auf begrenzte Bereiche der Haut aufgegossen oder aufgespritzt, wobei der Wirkstoff sich auf der Körperoberfläche verteilt.

Aufgießformulierungen werden hergestellt, indem der Wirkstoff in geeigneten hautverträglichen Lösungsmitteln oder Lösungsmittelgemischen gelöst, suspendiert oder emulgiert wird. Gegebenenfalls werden weitere Hilfsstoffe wie Farbstoffe, Antioxidantien, Lichtschutzmittel, Haftmittel zugefügt.

Als Lösungsmittel seien genannt: Wasser, Alkanole, Glycole, Polyethylenglycole, Polypropylenglycole, Glycerin, aromatische Alkohole wie Benzylalkohol, Phenylethanol, Phenoxyethanol, Ester wie Essigester, Butylacetat, Benzylbenzoat, Ether wie Alkylenglykolalkylether wie Dipropylenglykohnonomethylether, Diethylenglykolmonobutylether, Ketone wie Aceton, Methylethylketon, aromatische und/oder aliphatische Kohlenwasserstoffe, pflanzliche oder synthetische Öle, DMF, Dimethylacetamid, N-Methylpyrrolidon, 2-Dimethyl-4-oxy-methylen-1,3-dioxolan.

Farbstoffe sind alle zur Anwendung am Tier zugelassenen Farbstoffe, die gelöst oder suspendiert sein können.

Hilfsstoffe sind auch spreitende Öle wie Isopropylmyristat, Dipropylenglykolpelargonat, Silikonöle, Fettsäureester, Triglyceride, Fettalkohole.

Antioxidantien sind Sulfite oder Metabisulfite wie Kaliummetabisulfit, Ascorbinsäure, Butylhydroxytoluol, Butylhydroxyanisol, Tocopherol.

Lichtschutzmittel sind z.B. Stoffe aus der Klasse der Benzophenone oder Novantisolsäure.

Haftmittel sind z.B. Cellulosederivate, Stärkederivate, Polyacrylate, natürliche Polymere wie Alginate, Gelatine.

Emulsionen sind entweder vom Typ Wasser in Öl oder vom Typ Öl in Wasser.

Sie werden hergestellt, indem man den Wirkstoff entweder in der hydrophoben oder in der hydrophilen Phase löst und diese unter Zuhilfenahme geeigneter Emulgatoren und gegebenenfalls weiterer Hilfsstoffe wie Farbstoffe, resorptionsfördemde Stoffe, Konservierungsstoffe, Antioxidantien, Lichtschutzmittel, viskositätserhöhende Stoffe, mit dem Lösungsmittel der anderen Phase homogenisiert.

Als hydrophobe Phase (Öle) seien genannt: Paraffinöle, Silikonöle, natürliche Pflanzenöle wie Sesamöl, Mandelöl, Rizinusöl, synthetische Triglyceride wie Capryl/Caprinsäurebiglycerid, Triglyceridgemisch mit Pflanzenfettsäuren der Kettenlänge C₈₋₁₂ oder anderen speziell ausgewählten natürlichen Fettsäuren, Partialglyceridgemische gesättigter oder ungesättigter eventuell auch hydroxylgruppenhaltiger Fettsäuren, Mono- und Diglyceride der C₈/₁₀-Fettsäuren.

Fettsäureester wie Ethylstearat, Di-n-butyryl-adipat, Laurinsäurehexylester, Dipropylen-glykolpelargonat, Ester einer verzweigten Fettsäure mittlerer Kettenlänge mit gesättigten Fettalkoholen der Kettenlänge C₁₆-C₁₈, Isopropylmyristat, Isopropylpalmitat, Capryl/Caprinsäureester von gesättigten Fettalkoholen der Kettenlänge C₁₂-C₁₈, Isopropylstearat, Ölsäureoleylester, Ölsäuredecylester, Ethyloleat, Milchsäureethylester, wachsartige Fettsäureester wie Dibutylphthalat, Adipinsäurediisopropylester, letzterem verwandte Estergemische u.a. Fettalkohole wie Isotridecylalkohol, 2-Octyldodecanol, Cetylstearyl-alkohol, Oleylalkohol. Fettsäuren wie z.B. Ölsäure und ihre Gemische.

Als hydrophile Phase seien genannt:
Wasser, Alkohole wie z.B. Propylenglycol, Glycerin, Sorbitol und ihre Gemische.
Als Emulgatoren seien genannt: nichtionogene Tenside, z.B. polyoxyethyliertes Rizinusöl, polyoxyethyliertes Sorbitanmonooleat, Sorbitanmonostearat, Glycerinmonostearat, Polyoxyethylstearat, Alkylphenolpolyglykolether;
ampholytische Tenside wie Di-Na-N-lauryl-β-iminodipropionat oder Lecithin;
anionaktive Tenside, wie Na-Laurylsulfat, Fettalkoholethersulfate, Mono/Dialkylpolyglykoletherorthophosphorsäureestermonoethanolaminsalz; kationaktive Tenside wie Cetyltrimethylammoniumchlorid.

Als weitere Hilfsstoffe seien genannt: Viskositätserhöhende und die Emulsion stabilisierende Stoffe wie Carboxymethylcellulose, Methylcellulose und andere Cellulose- und Stärke-Derivate, Polyacrylate, Alginate, Gelatine, Gummi-arabicum, Polyvinylpyrrolidon, Polyvinylalkohol, Copolymere aus Methylvinylether und Maleinsäureanhydrid, Polyethylenglykole, Wachse, kolloidale Kieselsäure oder Gemische der aufgeführten Stoffe.

Suspensionen werden hergestellt, indem man den Wirkstoff in einer Trägerflüssigkeit gegebenenfalls unter Zusatz weiterer Hilfsstoffe wie Netzmittel, Farbstoffe, resorptionsfördernde Stoffe, Konservierungsstoffe, Antioxidantien, Lichtschutzmittel suspendiert.

Als Trägerflüssigkeiten seien alle homogenen Lösungsmittel und Lösungsmittelgemische genannt.

Als Netzmittel (Dispergiermittel) seien die weiter oben angegebenen Tenside genannt.

Als weitere Hilfsstoffe seien die weiter oben angegebenen genannt.

Halbfeste Zubereitungen zur dermalen Verabreichung unterscheiden sich von den oben beschriebenen Suspensionen und Emulsionen nur durch ihre höhere Viskosität.

Zur Herstellung fester Zubereitungen wird der Wirkstoff mit geeigneten Trägerstoffen gegebenenfalls unter Zusatz von Hilfsstoffen vermischt und in die gewünschte Form gebracht.

Als Trägerstoffe seien genannt alle physiologisch verträglichen festen Inertstoffe. Als solche dienen anorganische und organische Stoffe. Anorganische Stoffe sind z.B. Kochsalz, Carbonate wie Calciumcarbonat, Hydrogencarbonate, Aluminiumoxide, Kieselsäuren, Tonerden, gefälltes oder kolloidales Siliciumdioxid, Phosphate.

Hilfsstoffe sind Konservierungsstoffe, Antioxidantien, Farbstoffe, die bereits weiter oben aufgeführt worden sind.

Weitere geeignete Hilfsstoffe sind Schmier- und Gleitmittel wie z.B. Magnesiumstearat, Stearinsäure, Talkum, Bentonite.

Weiterhin ist es wünschenswert, dass ein solches Schutzmittel auch noch nach längerem Wasserkontakt, beispielsweise beim Schwimmen, Kleiderwaschen oder Fischen, noch eine ausreichende Schutzwirkung zeigt. Zu diesem Zweck können die erfindungsgemäßen Mittel auch wasserabweisende bzw. wasserfeste Substanzen enthalten.

Geeignete wasserfeste Substanzen wurden bisher schon in Sonnenschutzmitteln eingesetzt, die den Benutzer gegen die UV-Strahlung der Sonne schützen sollen (z.B. US 5 518 712 und US 4 810 489). Das Ziel war dabei, den Sonnenschutz auch aufrecht zu erhalten wenn der Benutzer schwimmen war oder heftig schwitzt etc. Sonnenschutzmittel enthaltend solche wasserfesten bzw. wasserabweisenden Substanzen und Insektenrepellentien sind bereits bekannt (US 5 716 602). Bisher wurden jedoch noch keine Mittel, enthaltend Anthelminthika beschrieben.

Es können dementsprechend auch wasserfeste Substanzen im erfindungsgemäßen Mittel enthalten sein. Dies können fettlösliche, wasserunlösliche Stoffe sein sowie Verbindungen welche die Haftung des Mittels auf der Haut erhöhen.

In Hautschutzprodukten könnten als wasserfeste Bestandteile beispielsweise 1 bis 50 Gew.-% eines Polymers wie Polyinylpyrrolidone, Polyacrylate, Silicone etc. enthalten sein.

Die Mittel zur topischen Anwendung können als Spray, Lösung, Creme, Salbe oder schicht- bzw. filmbildende Mittel, nach den zur Herstellung von Kosmetika bekannten Verfahren (Schrader, K. (1979) Grundlagen und Rezepturen der Kosmetika. Dr. Alfred Hüthig Verlag, Heidelberg), formuliert werden.

Zur Anwendung werden die erfindungsgemäßen Formulierungen in verbrauchergerechter Menge gleichmäßig und lückenlos abdeckend auf die Haut aufgetragen.

Die erfindungsgemäßen Mittel sind selbstverständlich auch zur Anwendung am Tier geeignet, um die Infektion der Tiere mit Parasiten dieser Gattungen zu verhindern. Die Mittel können bei Hobbytieren, wie beispielsweise Hunden und Katzen und bei Nutztieren, beispielsweise Rindern, Schweinen, Schafen u. a. angewendet werden.

Bei der Anwendung der erfindungsgemäßen Mittel werden im allgemeinen 0,03 bis 1 mg, bevorzugt 0,03 bis 0,1 mg und besonders bevorzugt 0,04 bis 0,06 mg des Wirkstoffes pro Quadratzentimeter Haut aufgebracht. Dadurch wird ein prophylaktischer Schutz gegen hautdurchdringende Würmer bzw. deren Vorstadien erreicht. Während eines längeren Aufenthaltes im Wasser ist der Wirkstoff wiederholt aufzutragen.

Die erfindungsgemäßen Mittel werden durch die folgenden Beispiele illustriert ohne sie jedoch einzuschränken.

### Biologisches Beispiel

### Wirksamkeit gegen Schistosoma mansoni-Cercarien

### [500 µl/l Endkonzentration der Wirkstoffe]

Zur Infektion wurden Schnecken (*Biomphalaria glabrata*) mit jeweils 8 Miracidien in in 10 ml Wasser über Nacht inkubiert. Cercarien wurden etwa 6 bis 9 Wochen nach Infektion gewonnen, indem die im Dunkeln gehaltenen Schnecken mit Licht bestrahlt und die danach ausschwärmenden Cercarien innerhalb von 2 Stunden gesammelt wurden.

Den Versuchsansätzen wurde soviel Cercarien-haltiges Wasser zugesetzt (1 bzw. 2 ml, siehe unten), daß jeder Ansatz etwa 100 bis 150 Cercarien enthielt.

5 µl Wirkstoff wurden mit 25 µl PEG300 gründlich vermischt. Anschließend wurde 9 ml Aquarienwasser zugesetzt und der Ansatz heftig geschüttelt. Nach (zeitversetzter) Zugabe von 1 ml Cercarien-Suspension wurde jeweils ab sofort mit der Stereolupe das Überleben der Cercarien beobachtet. Zur Einteilung der Wirksamkeit der Wirkstoffe wurde folgende Einteilung verwendet : 0 = keine Wirkung über die gesamte Versuchsdauer von 120 Minuten; 1 = schwache Wirkung (Cercarien weisen eine stark verringerte Beweglichkeit auf); 2 = gute Wirkung (Cercarien sind nur noch leicht beweglich und gekrümmt); 3 = volle Wirksamkeit (Cercarien sind vollkommen regungslos)

### Bewertung für verschiedene erfindungsgemäße Verbindungen:

| | |
|---|---|
| Verbindung | Bewertung |
| 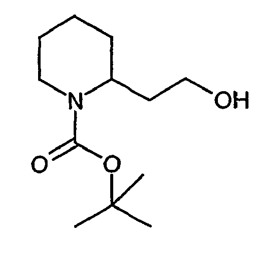 | 3 |
| 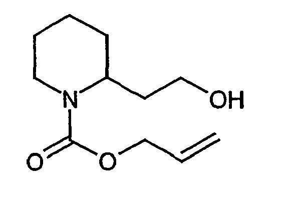 | 3 |
| 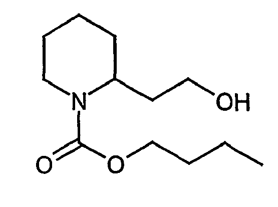 | 3 |
| 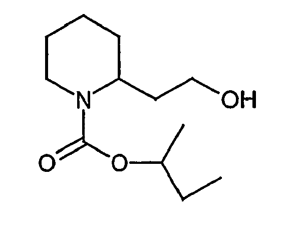 | 2 |
| 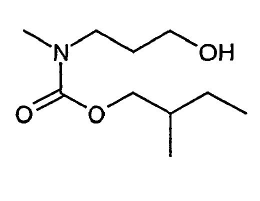 | 1 |
| 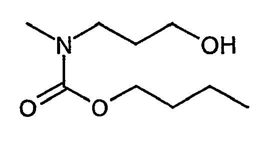 | 1 |
| 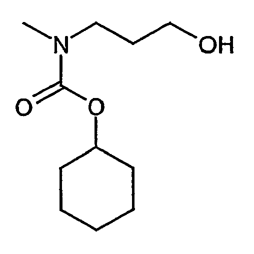 | 1 |
| 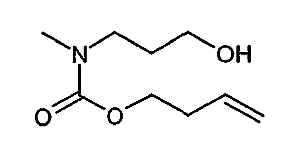 | 2 |

## Patentansprüche

1. Mittel zur Anwendung in einem Verfahren zur Abwehr von helminthischen Parasiten, **gekennzeichnet durch** einen Gehalt an mindestens einer Verbindung der Formel (I) 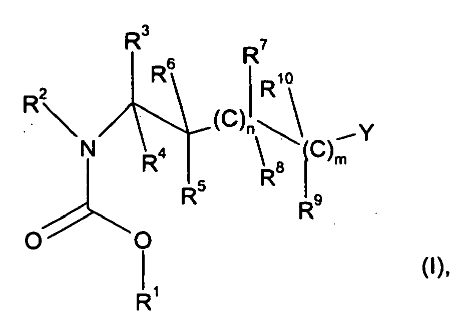 in welcher
Y für Wasserstoff, gegebenenfalls substituiertes Alkyl oder für den Rest O-X steht,
X für Wasserstoff, COR¹¹, COOR¹² oder R¹³ steht,
R¹ für einen gegebenenfalls substituierten Alkyl-, Alkenyl-, Alkinyl-, Cycloalkyl- oder Cycloalkenylrest steht,
R², R¹¹, R¹² und R¹³ gleich oder verschieden sind und für gegebenenfalls substituierte Alkyl- oder Alkenylreste stehen,
R³ bis R¹⁰ gleich oder verschieden sind und für Wasserstoff oder für gegebenenfalls substituierte Alkylreste stehen und wobei R² und R³ oder R³ und R⁷ oder R³ und R⁵ oder R⁵ und R⁷ gemeinsam mit den Atomen, an welche sie gebunden sind, auch gegebenenfalls substituierten monocyclischen Ring bilden können
und
n und m gleich oder verschieden sind und 0 oder 1 bedeuten.

2. Mittel zur Anwendung in einem Verfahren zur Abwehr von helminthischen Parasiten gemäß Anspruch 1, **dadurch gekennzeichnet, dass** sie mindestens eine Verbindung der Formel (I) enthalten,
Y für Wasserstoff, C₁-C₆-Alkyl oder für den Rest O-X steht,
X für Wasserstoff, COR¹¹ oder R¹³ steht,
R¹ für C₃-C₇-Cycloalkyl, C₃-C₇-Ccloalkenyl, C₁-C₂-Alkyl-C₃-C₇-Cycloalkyl, C₁-C₂-Alkyl-C₃-C₇-Cycloalkenyl, wobei die Cycloalkyl- oder Cycloalkenylringe der vorgenannten Reste gegebenenfalls bis zu dreifach durch C₁-C₆-Alkyl oder durch eine C₁-C₆-Dialkylenbrücke substituiert sind, oder
R¹ für C₁-C₇-Alkyl, C₂-C₇-Alkenyl oder C₂-C₇-Alkinyl steht,
R², R¹¹, R¹³ gleich oder verschieden sind und für C₁-C₆-Alkyl stehen,
R³ bis R⁸ gleich oder verschieden sind und für Wasserstoff oder C₁-C₆-Alkyl stehen, wobei R² und R³ oder R³ und R⁷ oder R³ und R⁵ oder R⁵ und R⁷ gemeinsam mit den Atomen, an welche sie gebunden sind, auch einen 5- oder 6-gliedrigen monocyclischen Ring bilden können und
N für 1 und m für 0 steht.

3. Verwendung von Verbindungen der Formel (I) gemäß Anspruch 1 für die Herstellung von Mitteln zur Abwehr von helminthischen Parasiten.

4. Verwendung nach Anspruch 3, **dadurch gekennzeichnet, dass** man Verbindungen der Formel (I) gemäß Anspruch 1 mit Streckmitteln und/oder oberflächenaktiven Mitteln vermischt.

## Claims

1. Compositions for use in a method for deterring helmintic parasites, **characterized in that** they comprise at least one compound of the formula (I) 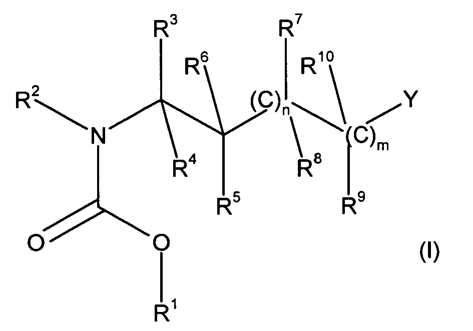 in which
Y represents hydrogen, optionally substituted alkyl or the radical O-X,
X represents hydrogen, COR¹¹, COOR¹² or R¹³,
R¹ represents an optionally substituted alkyl, alkenyl, alkinyl, cycloalkyl or cycloalkenyl radical,
R², R¹¹, R¹² and R¹³ are identical or different and represent optionally substituted alkyl or alkenyl radicals,
R³ to R¹⁰ are identical or different and represent hydrogen or represent optionally substituted alkyl radicals, where R² and R³ or R³ and R⁷ or R³ and R⁵ or R⁵ and R⁷ together with the atoms to which they are attached may also form an optionally substituted monocyclic ring
and
n and m are identical or different and are 0 or 1.

2. Compositions for use in a method for deterring helmintic parasites according to Claim 1,
**characterized in that** they comprise at least one compound of the formula (I)
in which
Y represents hydrogen, C₁-C₆-alkyl or the radical O-X,
X represents hydrogen, COR¹¹ or R¹³,
R¹ represents C₃-C₇-cycloalkyl, C₃-C₇-cycloalkenyl, C₁-C₂-alkyl-C₃-C₇-cycloalkyl, C₁-C₂-alkyl-C₃-C₇-cycloalkenyl, where the cycloalkyl or cycloalkenyl rings of the abovementioned radicals are optionally substituted up to three times by C₁-C₆-alkyl or by a C₁-C₆-dialkylene bridge, or
R¹ represents C₁-C₇-alkyl, C₂-C₇-alkenyl or C₂-C₇-alkinyl,
R², R¹¹, R¹³ are identical or different and represent C₁-C₆-alkyl,
R³ to R⁸ are identical or different and represent hydrogen or C₁-C₆-alkyl, where R² and R³ or R³ and R⁷ or R³ and R⁵ or R⁵ and R⁷ together with the atoms to which they are attached may also form a 5- or 6-membered monocyclic ring and
n represents 1 and m represents 0.

3. Use of compounds of the formula (I) according to Claim 1 for preparing compositions for deterring helmintic parasites.

4. Use according to Claim 3, **characterized in that** compounds of the formula (I) according to Claim 1 are mixed with extenders and/or surfactants.

## Revendications

1. Composition pour utilisation dans un procédé de défense contre des parasites helminthiques, **caractérisée par** une teneur en au moins un composé de formule (I) 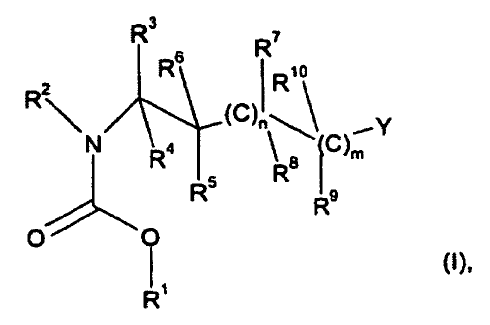 dans laquelle
Y représente un atome d'hydrogène, un groupe alkyle éventuellement substitué ou le radical O-X,
X représente un atome d'hydrogène, COR¹¹, COOR¹² ou R¹³,
R¹ représente un radical alkyle, alcényle, alcynyle, cycloalkyle ou cycloalcényle,
R², R¹¹, R¹² et R¹³ sont identiques ou différents et représentent des radicaux alkyle ou alcényle éventuellement substitués,
R³ à R¹⁰ sont identiques ou différents et représentent un atome d'hydrogène ou des radicaux alkyle éventuellement substitués, R² et R³ ou R³ et R⁷ ou R³ et R⁵ ou R⁵ et R⁷ pouvant également former ensemble avec les atomes auxquels ils sont liés un cycle monocyclique éventuellement substitué
et
n et m sont identiques ou différents et représentent 0 ou 1.

2. Composition pour utilisation dans un procédé de défense contre des parasites helminthiques selon la revendication 1, **caractérisée en ce qu'**elle contient au moins un composé de formule (I)
dans laquelle
Y représente un atome d'hydrogène, un groupe alkyle en C₁-C₆ ou le radical O-X,
X représente un atome d'hydrogène, COR¹¹ ou R¹³,
R¹ représente un radical cycloalkyle en C₃-C₇, cycloalcényle en C₃-C₇, alkyl (C₁-C₂)-cycloalkyle(C₃-C₇), alkyl (C₁-C₂)-cycloalcényle(C₃-C₇), les cycles cycloalkyle ou cycloalcényle des radicaux précités étant éventuellement jusqu'à trois fois substitués par alkyle en C₁-C₆ ou par un pont dialkylène en C₁-C₆, ou
R¹ représente un groupe alkyle en C₁-C₇, alcényle en C₂-C₇ ou alcynyle en C₂-C₇,
R², R¹¹, R¹³ sont identiques ou différents et représentent des radicaux alkyle en C₁-C₆,
R³ à R⁸ sont identiques ou différents et représentent un atome d'hydrogène ou un radical alkyle en C₁-C₆, R² et R³ ou R³ et R⁷ ou R³ et R⁵ ou R⁵ et R⁷ pouvant également former ensemble, avec les atomes auxquels ils sont liés, un cycle monocyclique à 5 ou 6 chaînons et
n représente 1 et m représente 0.

3. Utilisation des composés de formule (I) selon la revendication 1, pour la préparation de compositions destinées à la défense contre des parasites helminthiques.

4. Utilisation selon la revendication 3, **caractérisée en ce qu'**on mélange des composés de formule (I) selon la revendication 1 avec des diluants et/ou des agents tensioactifs.
